# EUROPEAN PATENT SPECIFICATION

(11) **EP 3 865 070 B1**
(45) Date of publication and mention of the grant of the patent: **31.07.2024**
(21) Application number: 19870365.4
(22) Date of filing: 17.09.2019
(51) Int. Cl.: A61B 8/00

(54) **ULTRASOUND DIAGNOSIS DEVICE AND ULTRASOUND DIAGNOSIS DEVICE CONTROL METHOD**
ULTRASCHALLDIAGNOSEVORRICHTUNG UND VERFAHREN ZUR STEUERUNG DER ULTRASCHALLDIAGNOSEVORRICHTUNG
DISPOSITIF DE DIAGNOSTIC À ULTRASONS ET PROCÉDÉ DE COMMANDE DE DISPOSITIF DE DIAGNOSTIC À ULTRASONS

(30) Priority: 12.10.2018 JP 2018193250
(43) Date of publication of application: 18.08.2021
(73) Proprietor: FUJIFILM Corporation, Tokyo 106-8620 (JP)
(72) Inventor: TSUTAOKA Takuya, Ashigarakami-gun, Kanagawa 258-8538 (JP)
(74) Representative: Kudlek, Franz Thomas
(86) International application number: PCT/JP2019/036276
(87) International publication number: WO 2020/075449

(56) References cited:
- EP-A1- 3 308 713
- WO-A1-2018/185021
- JP-A- 2009 512 532
- JP-A- 2016 195 748
- US-A1- 2016 367 218
- US-A1- 2017 202 503
- US-A1- 2018 064 413
- JOENSSON I M ET AL: "Transabdominal Ultrasound of Rectum as a Diagnostic Tool in Childhood Constipation", JOURNAL OF UROLOGY, LIPPINCOTT WILLIAMS & WILKINS, BALTIMORE, MD, US, vol. 179, no. 5, 1 May 2008 (2008-05-01), pages 1997 - 2002, XP022594509, ISSN: 0022-5347, [retrieved on 20080320], DOI: 10.1016/J.JURO.2008.01.055

## Description

### BACKGROUND OF THE INVENTION

### 1. Field of the Invention

The present invention relates to an ultrasound diagnostic apparatus and a control method of an ultrasound diagnostic apparatus, and more particularly, to an ultrasound diagnostic apparatus and a control method of an ultrasound diagnostic apparatus which are used to examine the lower abdomen of a subject.

### 2. Description of the Related Art

Hitherto, in a medical field, an ultrasound diagnostic apparatus using an ultrasound image has been put to practical use. Generally, this kind of ultrasound diagnostic apparatus includes an ultrasound probe having a built-in transducer array and an apparatus body connected to the ultrasound probe, in which ultrasonic waves are transmitted from the ultrasound probe toward a subject, the ultrasound probe receives ultrasound echoes from the subject, and the apparatus body electrically processes the received signal to generate an ultrasound image.

The condition of stool or the like of the subject has been evaluated by examining the lower abdomen of the subject by using such an ultrasound diagnostic apparatus. For example, JP2016-195748A discloses an ultrasound diagnostic apparatus that analyzes the ultrasound echoes from the large intestine of the subject, thereby automatically evaluating the condition of stool inside the large intestine.
JOENSSON et al.: "Transabdominal Ultrasound of Rectum as a Diagnostic Tool in Childhood Constipation", JOURNAL OF UROLOGY, LIPPINCOTT WILLIAMS & WILKINS, BALTIMORE, MD, US, vol. 179, no. 5, 1 May 2008 (2008-05-01), pages 1997-2002, XP022594509, ISSN: 0022-5347, DOI: 10.1016/J.JURO.2008.01.055 describes a scientific study, in which it was tested whether transverse rectal diameter measured by ultrasound could identify rectal impaction, investigated whether transverse diameter is enlarged in constipated children compared to healthy children and evaluated transverse diameter during treatment of constipation.
US 2016/367218 A1 discloses an apparatus for measuring an amount of urine in a bladder. The apparatus includes a transducer, a switch for selecting one of operational modes, which include a bladder position check mode and a urine amount measurement mode, and a central control unit for operating the apparatus according to the operational mode selected by the switch. The central control unit in the bladder position check mode repeats sequentially predetermined operations until the urine amount measurement mode is selected, the predetermined operations include performing a preliminary scan to a single scan plane related to an aiming direction of the transducer, detecting a center point of the bladder in a ultrasound image, and representing a predetermined mark at the center point of the bladder in the displayed ultrasound image in order to indicate visually a center point of the bladder.

### SUMMARY OF THE INVENTION

Incidentally, upon the examination of the lower abdomen of the subject using the ultrasound diagnostic apparatus, it is desirable for the user to examine the subject within a limited time in order to reduce the burden on the subject. Further, in recent years, a portable ultrasound diagnostic apparatus has been developed, and the examination of the lower abdomen of the subject using the ultrasound diagnostic apparatus has been performed even in a field of home medical care. In particular, in such a field of home medical care, there are many users who are unfamiliar with the examination of the lower abdomen using the ultrasound diagnostic apparatus. The user needs to perform the examination of the subject without omission within a limited time, and thus it has been required to reduce manually setting and operating the ultrasound diagnostic apparatus so that a low-skilled user also easily performs the ultrasonic examination.

However, in a case of examining the subject by using the ultrasound diagnostic apparatus disclosed in JP2016-195748A, the user needs to manually set the transmission/reception conditions of the ultrasonic waves in the ultrasound diagnostic apparatus according to the site of the subject to be examined. Thus, there has been a problem that a lot of effort and time may be required, particularly in a case where the low-skilled user performs the examination. In addition, in a case of examining the lower abdomen of the subject by using the ultrasound diagnostic apparatus, not only the examination of the large intestine but also the examination of a plurality of sites, such as a bladder is continuously performed in many cases, and thus the user needs to manually set the transmission/reception conditions of the ultrasonic waves in the ultrasound diagnostic apparatus every time the site to be examined changes, which causes a problem that more effort and time may be required upon the examination.

The present invention has been made to solve such a conventional problem and an object thereof is to provide an ultrasound diagnostic apparatus and a control method of an ultrasound diagnostic apparatus which can reduce the effort of the user and can easily perform examination regardless of the skill level of the user.

In order to achieve the above object, an ultrasound diagnostic apparatus according to an aspect of the present invention comprises an ultrasound probe; an image acquisition unit that acquires an ultrasound image of a subject by performing transmission and reception of an ultrasound beam to and from the ultrasound probe in accordance with a predetermined transmission/reception condition; a display unit that displays the ultrasound image acquired by the image acquisition unit; a constipation evaluation unit that performs constipation evaluation based on the ultrasound image acquired by the image acquisition unit; and a urine volume measurement availability determination unit that determines whether or not urine volume measurement is available, from the ultrasound image acquired by the image acquisition unit in accordance with the transmission/reception condition which is used to perform the constipation evaluation by the constipation evaluation unit.

In addition, it is preferable that the ultrasound diagnostic apparatus further comprises a bladder extraction unit that extracts a bladder based on the ultrasound image acquired by the image acquisition unit, in which in a case where an ultrasound image from which the bladder is extracted by the bladder extraction unit is present in a series of the ultrasound images acquired by the image acquisition unit in accordance with the transmission/reception condition which is used to perform the constipation evaluation by the constipation evaluation unit, the urine volume measurement availability determination unit determines that the urine volume measurement is available.

In a case where the urine volume measurement availability determination unit determines that the urine volume measurement is available, the urine volume measurement availability determination unit may display the ultrasound image from which the bladder is extracted by the bladder extraction unit and a guidance display prompting a user to give an instruction on whether or not to carry out the urine volume measurement, on the display unit.

In this case, it is preferable that the ultrasound diagnostic apparatus further comprises an input unit that is used for the user to perform input operation.

In addition, the ultrasound diagnostic apparatus may further comprise a urine volume measurement unit that performs the urine volume measurement based on the ultrasound image from which the bladder is extracted by the bladder extraction unit.

In this case, in a case where the user gives the instruction on carrying out the urine volume measurement through the input unit, the urine volume measurement unit may measure a bladder diameter, based on an ultrasound image which includes a bladder having a largest area among a plurality of the ultrasound images from which the bladder is extracted by the bladder extraction unit, and display the ultrasound image which includes the bladder having the largest area and a measurement result of the bladder diameter, on the display unit.

A control method of an ultrasound diagnostic apparatus according to another aspect of the present invention comprises acquiring an ultrasound image of a subject by performing transmission and reception of an ultrasound beam to and from the ultrasound probe in accordance with a predetermined transmission/reception condition; displaying the acquired ultrasound image; performing constipation evaluation based on the acquired ultrasound image; and determining whether or not urine volume measurement is available, from the ultrasound image acquired in accordance with the transmission/reception condition which is used to perform the constipation evaluation.

According to the present invention, the ultrasound diagnostic apparatus comprises the constipation evaluation unit that performs constipation evaluation based on the ultrasound image acquired by the image acquisition unit and a urine volume measurement availability determination unit that determines whether or not urine volume measurement is available, from the ultrasound image acquired by the image acquisition unit in accordance with the transmission/reception condition which is used to perform the constipation evaluation by the constipation evaluation unit. Therefore, the effort of the user can be reduced and the examination can be easily performed regardless of the skill level of the user.

### BRIEF DESCRIPTION OF THE DRAWINGS

Fig. 1 is a block diagram showing a configuration of an ultrasound diagnostic apparatus according to an embodiment of the present invention.
Fig. 2 is a block diagram showing an internal configuration of a reception unit according to the embodiment of the present invention.
Fig. 3 is a block diagram showing an internal configuration of an image generation unit according to the embodiment of the present invention.
Fig. 4 is a diagram schematically showing an example of a guidance display on urine volume measurement.
Fig. 5 is a diagram schematically showing a display example of a measurement result of a bladder diameter.
Fig. 6 is a flowchart showing an operation of the ultrasound diagnostic apparatus according to the embodiment of the present invention.

### DESCRIPTION OF THE PREFERRED EMBODIMENTS

Hereinafter, an embodiment of the present invention will be described with reference to the accompanying drawings.

### Embodiment

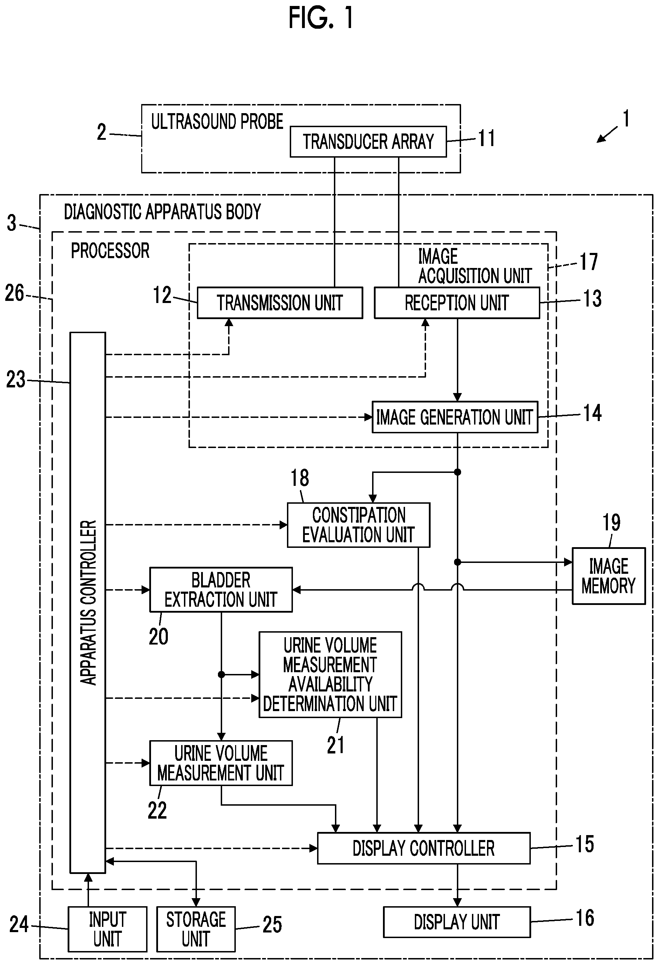

Fig. 1 shows the configuration of an ultrasound diagnostic apparatus 1. As shown in Fig. 1, the ultrasound diagnostic apparatus 1 has an ultrasound probe 2 and a diagnostic apparatus body 3, and the ultrasound probe 2 and the diagnostic apparatus body 3 are connected to each other. The ultrasound probe 2 comprises a transducer array 11. Here, the ultrasound probe 2 and the diagnostic apparatus body 3 may be connected wiredly or wirelessly to each other.

The diagnostic apparatus body 3 comprises a transmission unit 12 and a reception unit 13, and the transmission unit 12 and the reception unit 13 are connected to the transducer array 11 of the ultrasound probe 2. Further, the reception unit 13 is sequentially connected to the image generation unit 14, the display controller 15, and the display unit 16. Here, the transmission unit 12, the reception unit 13, and the image generation unit 14 constitute an image acquisition unit 17. In addition, a constipation evaluation unit 18 and an image memory 19 are connected to the image generation unit 14. The display controller 15 is connected to the constipation evaluation unit 18, and the bladder extraction unit 20 is connected to the image memory 19. Further, a urine volume measurement availability determination unit 21 and a urine volume measurement unit 22 are connected to the bladder extraction unit 20, and the display controller 15 is connected to the urine volume measurement availability determination unit 21 and the urine volume measurement unit 22.

Furthermore, an apparatus controller 23 is connected to the transmission unit 12, the reception unit 13, the image generation unit 14, the display controller 15, the constipation evaluation unit 18, the bladder extraction unit 20, the urine volume measurement availability determination unit 21, and the urine volume measurement unit 22. An input unit 24 and a storage unit 25 are connected to the apparatus controller 23. Here, the apparatus controller 23 and the storage unit 25 are connected to each other such that information can be exchanged in both directions.

In addition, a display controller 15, an image acquisition unit 17, a constipation evaluation unit 18, a bladder extraction unit 20, a urine volume measurement availability determination unit 21, a urine volume measurement unit 22, and the apparatus controller 23 constitute the processor 26.

The transducer array 11 of the ultrasound probe 2 shown in Fig. 1 has a plurality of transducers arranged one-dimensionally or two-dimensionally. Each of the transducers transmits ultrasonic waves in accordance with a drive signal supplied from the transmission unit 12, receives ultrasound echoes from the subject, and outputs the received signal. For example, each transducer is formed by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

The transmission unit 12 of the image acquisition unit 17 includes, for example, a plurality of pulse generators, and the transmission unit 12 adjusts the amount of delay of each drive signal based on a transmission delay pattern selected according to a control signal from the apparatus controller 23 in accordance with the preset transmission condition of the ultrasonic waves, and supplies the drive signals to the plurality of transducers so that the ultrasonic waves transmitted from the plurality of transducers of the transducer array 11 form an ultrasound beam. In this manner, in a case where a pulsed or continuous-wave voltage is applied to the electrodes of the plurality of transducers of the transducer array 11, the piezoelectric body expands and contracts to generate pulsed or continuous-wave ultrasonic waves from each transducer. From the combined wave of these ultrasonic waves, the ultrasound beam is formed.

The transmitted ultrasound beam is reflected by a target, for example, a site of the subject, and propagates toward the transducer array 11 of the ultrasound probe 2. The ultrasound echoes propagating toward the transducer array 11 in this manner are received by each transducer constituting the transducer array 11. In this case, each transducer constituting the transducer array 11 expands and contracts by receiving the propagating ultrasound echoes to generate an electrical signal, and outputs the electrical signal to the reception unit 13.

The reception unit 13 of the image acquisition unit 17 processes the received signal output from the transducer array 11, based on the control signal from the apparatus controller 23 in accordance with the reception condition of the preset ultrasonic waves.

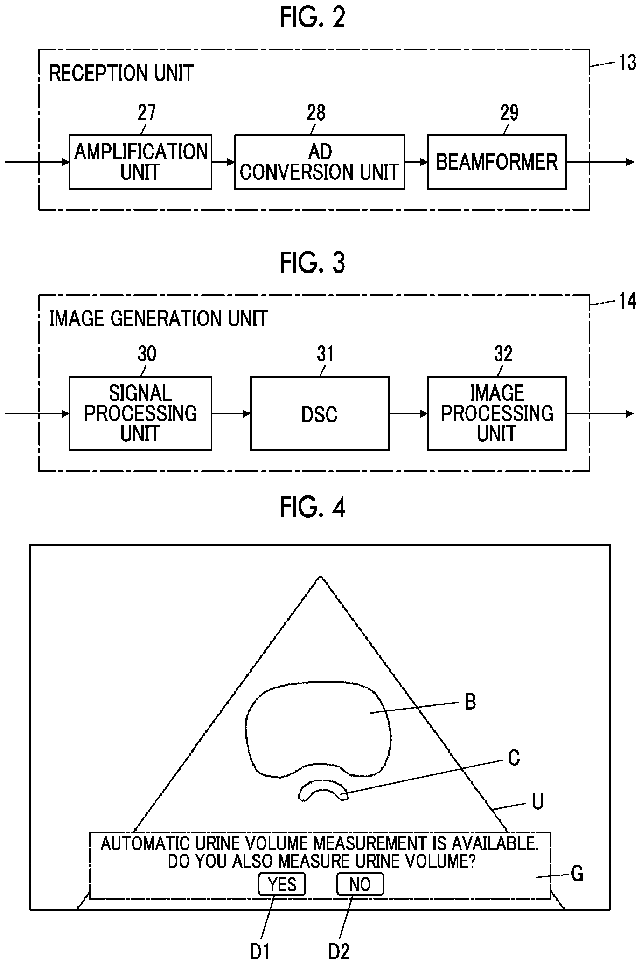

As shown in Fig. 2, the reception unit 13 has a configuration in which an amplification unit 27, an analog digital (AD) conversion unit 28, and a beamformer 29 are connected to one another in series. The amplification unit 27 amplifies the received signal received from each transducer constituting the transducer array 11, and transmits the amplified received signal to the AD conversion unit 28. The AD conversion unit 28 converts the received signal transmitted from the amplification unit 27 into digital data, and sends the data to the beamformer 29. Based on a reception delay pattern selected according to the control signal from the apparatus controller 23, the beamformer 29 performs reception focusing processing in which addition (phasing addition) is performed by giving delays to respective pieces of data according to a set sound speed. Through the reception focusing processing, a sound ray signal in which the focus of the ultrasound echo is narrowed on a fixed scanning line is generated. The sound ray signal generated in this manner is sent to the image generation unit 14.

As shown in Fig. 3, the image generation unit 14 of the image acquisition unit 17 has a configuration in which a signal processing unit 30, a digital scan converter (DSC) 31, and an image processing unit 32 are connected to one another in series. The signal processing unit 30 corrects the attenuation of the generated sound ray signal, which is caused by the propagation distance according to the depth of the reflection position of the ultrasonic wave, and then performs envelope detection processing to generate a B-mode image signal representing a tissue in a subject. The B-mode image signal generated in this manner is output to the DSC 31.

The DSC 31 of the image generation unit 14 raster-converts the B-mode image signal into an image signal according to a normal television signal scanning method to generate an ultrasound image. The image processing unit 32 of the image generation unit 14 performs various kinds of necessary image processing such as brightness correction, gradation correction, sharpness correction, and color correction on the image data obtained by the DSC 31, and then outputs the ultrasound image to the display controller 15, the constipation evaluation unit 18, and the image memory 19.

The constipation evaluation unit 18 of the processor 26 performs constipation evaluation based on the ultrasound image acquired by the image acquisition unit 17. Specifically, in a case where the image acquisition unit 17 acquires the ultrasound image in which the rectum of the subject is captured, the constipation evaluation unit 18 performs image analysis on the ultrasound image to evaluate, for example, whether the stool present in the rectum of the subject is loose stool or hard stool. In addition, in a case where the image acquisition unit 17 continuously acquires a plurality of ultrasound images, the constipation evaluation unit 18 performs the constipation evaluation by using a series of the ultrasound images.

The image memory 19 of the diagnostic apparatus body 3 stores the series of ultrasound images from which the constipation evaluation is performed by the constipation evaluation unit 18. As the image memory 19, for example, recording media such as a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), or a server may be used.

The bladder extraction unit 20 of the processor 26 performs image analysis to extract the bladder from the series of ultrasound images stored in the image memory 19. Here, the ultrasound image stored in the image memory 19 is an ultrasound image which is acquired by the image acquisition unit 17 for constipation evaluation and includes the rectum, but usually, the ultrasound image also includes the bladder because the bladder is anatomically present above the rectum. In addition, since the transmission/reception condition of the ultrasonic wave for imaging the rectum is usually within the range of the transmission/reception condition of the ultrasonic wave for imaging the bladder, the transmission/reception condition of the ultrasonic wave for imaging the rectum is also applied to imaging the bladder. Note that, examples of the transmission/reception conditions of the ultrasonic waves include the transmission/reception focus condition of the ultrasonic wave and the signal amplification factor in the amplification unit 27, that is, the gain condition.

In a case of extracting the bladder from the ultrasound image, the bladder extraction unit 20 stores, for example, typical pattern data in advance as a template, calculates the similarity to the pattern data while searching the image with the template, and thereby can extract the bladder on the assumption that the bladder is present at the place where the similarity is equal to or more than a threshold value, or maximum.

In addition to the simple template matching, in order to calculate the similarity, for example, a machine learning method described in Csurka et al.: Visual Categorization with Bags of Keypoints, Proc. of ECCV Workshop on Statistical Learning in Computer Vision, pp.59 to 74 (2004), a general image recognition method using deep learning described in Krizhevsk et al.: ImageNet Classification with Deep Convolutional Neural Networks, Advances in Neural Information Processing Systems 25, pp.1106 to 1114 (2012) may be used.

In a case where the ultrasound images from which the bladders are extracted are present in the series of ultrasound images stored in the image memory 19, the bladder extraction unit 20 sends information indicating that the bladders are extracted to the urine volume measurement availability determination unit 21.

In addition, in a case of extracting the bladders from the series of ultrasound images stored in the image memory 19, the bladder extraction unit 20 calculates the area of each of the extracted bladders and sends the ultrasound image which includes the bladder having the largest area to the urine volume measurement availability determination unit 21 and the urine volume measurement unit 22.

The urine volume measurement availability determination unit 21 of the processor 26 determines whether or not urine volume measurement following the constipation evaluation is available without change, from the ultrasound image acquired by the image acquisition unit 17 in accordance with the transmission/reception condition of the ultrasonic wave which is used to perform the constipation evaluation by the constipation evaluation unit 18. For example, in a case where the ultrasound images from which the bladders are extracted by the bladder extraction unit 20 are present in the series of ultrasound images acquired by the image acquisition unit 17 in accordance with the transmission/reception condition which is used to perform the constipation evaluation by the constipation evaluation unit 18, that is, the series of ultrasound images stored in the image memory 19, the urine volume measurement availability determination unit 21 determines that the urine volume measurement following the constipation evaluation is available without change.

Further, in a case where the urine volume measurement availability determination unit 21 determines that the urine volume measurement is available, as shown in Fig. 4, the urine volume measurement availability determination unit 21 may display the ultrasound image U from which the bladder B is extracted by the bladder extraction unit 20 and a guidance display G prompting a user to give an instruction on whether or not to carry out the urine volume measurement, on the display unit 16. The ultrasound image U includes a bladder B having the largest area among the bladders B extracted by the bladder extraction unit 20. Here, the guidance display G is displayed on the display unit 16 by text, an image, or the like, but in the example shown in Fig. 4, the guidance display G includes text "Automatic urine volume measurement is available. Do you also measure urine volume?", a first button D1 displayed as "YES", and a second button D2 displayed as "NO".

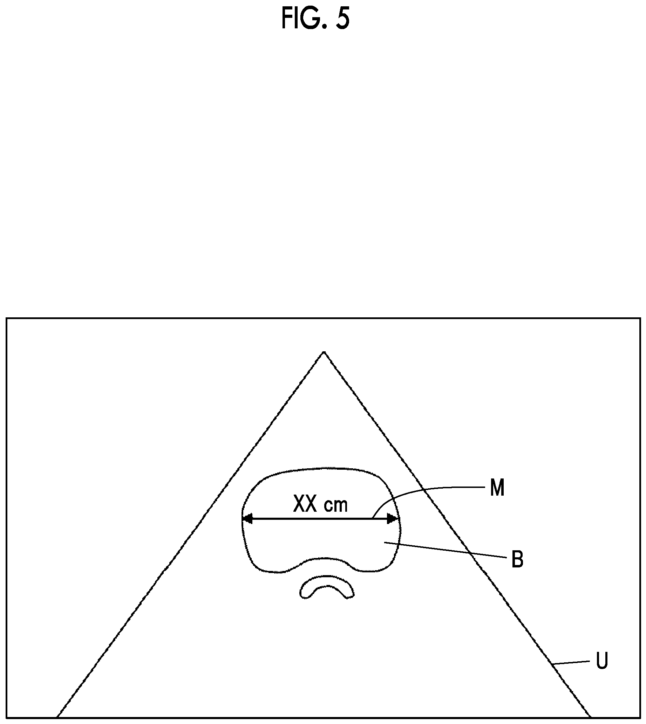

The urine volume measurement unit 22 of the processor 26 performs the urine volume measurement based on the ultrasound image U from which the bladder B is extracted by the bladder extraction unit 20. For example, in a case where the user gives the instruction on carrying out the urine volume measurement through the input unit 24, the urine volume measurement unit 22 measures a bladder diameter, based on an ultrasound image which includes a bladder B having the largest area among the plurality of ultrasound images from which the bladder is extracted by the bladder extraction unit 20. Further, for example, as shown in Fig. 5, the urine volume measurement unit 22 displays the ultrasound image U which includes the bladder B having the largest area and the measurement result M of the bladder diameter, on the display unit 16. In the example shown in Fig. 5, as the measurement result M, the text "XX cm" indicating the horizontal length of the bladder B and the measurement line indicating the horizontal length of the bladder B are displayed.

Under the control of the apparatus controller 23, the display controller 15 of the processor 26 performs predetermined processing on the ultrasound image acquired by the image acquisition unit 17, and causes the display unit 16 to display the ultrasound image.

The display unit 16 of the diagnostic apparatus body 3 displays an image under the control of the display controller 15, and examples thereof include a display device such as a liquid crystal display (LCD) and an organic electroluminescence display (organic EL display).

The apparatus controller 23 of the processor 26 controls each unit of the diagnostic apparatus body 3 based on the program stored in advance in the storage unit 25 or the like and the user's operation through the input unit 24.

The input unit 24 of the diagnostic apparatus body 3 is used for the user to perform an input operation, and may include a keyboard, a mouse, a trackball, a touch pad, a touch panel, and the like.

The storage unit 25 of the diagnostic apparatus body 3 stores an operation program or the like of the diagnostic apparatus body 3, and as the storage unit 25, for example, recording media such as an HDD, an SSD, an FD, an MO disc, an MT, a RAM, a CD, a DVD, an SD card and a USB memory, or a server may be used, as in the image memory 19.

Here, in the diagnostic apparatus body 3, the processor 26 having the display controller 15, the image acquisition unit 17, the constipation evaluation unit 18, the bladder extraction unit 20, the urine volume measurement availability determination unit 21, the urine volume measurement unit 22, and the apparatus controller 23 may be configured by a central processing unit (CPU) and a control program for causing the CPU to execute various kinds of processing, by using a field programmable gate array (FPGA), a digital signal processor (DSP), an application specific integrated circuit (ASIC), or other integrated circuits (IC), or by combination thereof.

Alternatively, the display controller 15, the image acquisition unit 17, the constipation evaluation unit 18, the bladder extraction unit 20, the urine volume measurement availability determination unit 21, the urine volume measurement unit 22, and the apparatus controller 23 of the processor 26 may be configured by being integrated partially or entirely into one CPU.

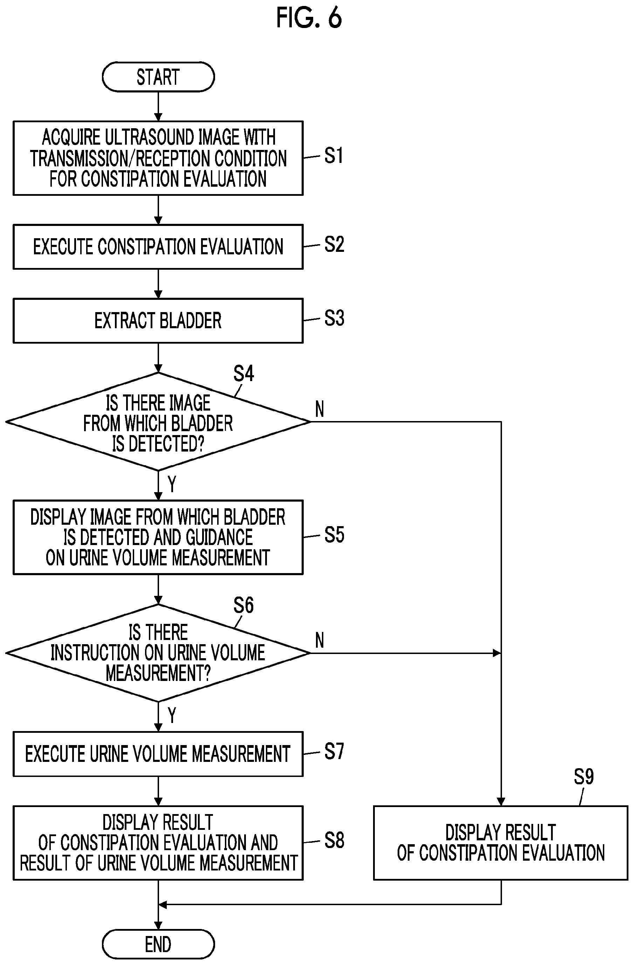

Next, the operation of the ultrasound diagnostic apparatus 1 according to the embodiment of the present invention will be described with reference to the flowchart shown in Fig. 6.

First, in Step S1, the image acquisition unit 17 continuously acquires a plurality of ultrasound images with the transmission/reception conditions of the ultrasonic waves for constipation evaluation. In this case, a series of the ultrasound images acquired by the image acquisition unit 17 are stored in the image memory 19.

Next, in Step S2, the constipation evaluation unit 18 executes constipation evaluation using the ultrasound images acquired in Step S1. When the constipation evaluation is completed, the process proceeds to Step S3.

In Step S3, the bladder extraction unit 20 performs image analysis to perform processing of extracting the bladders B from the series of ultrasound images stored in the image memory 19 in Step S1. In this case, the bladder extraction unit 20 may extract the bladders B, for example, by performing so-called template matching on the series of ultrasound images stored in the image memory 19.

Further, in a case where ultrasound images U from which the bladders B are extracted are present in the series of ultrasound images, the bladder extraction unit 20 sends information indicating that the bladders B are extracted to the urine volume measurement availability determination unit 21. Furthermore, in a case where the ultrasound images U from which the bladders B are extracted are present in the series of ultrasound images, the bladder extraction unit 20 measures the area of each of the extracted bladders B and sends the ultrasound image U which includes the bladder B having the largest area to the urine volume measurement availability determination unit 21 and the urine volume measurement unit 22.

In subsequent Step S4, the urine volume measurement availability determination unit 21 determines whether or not the bladder B is extracted in Step S3. In this case, the urine volume measurement availability determination unit 21 receives the information indicating that the bladder B is extracted by the bladder extraction unit 20, thereby determining that the bladder B is extracted in Step S3. In a case where the urine volume measurement availability determination unit 21 determines in Step S4 that the bladder B is extracted, the process proceeds to Step S5.

In Step S5, the urine volume measurement availability determination unit 21 causes the display unit 16 to display, for example, as shown in Fig. 4, the ultrasound image U from which the bladder B is extracted and the guidance display G to the user for the urine volume measurement.

In Step S6, the apparatus controller 23 determines whether or not an instruction on the urine volume measurement is given by the user. For example, as shown in Fig. 4, in a case where the user presses the first button D1 indicating that the urine volume measurement is executed, through the input unit 24, the apparatus controller 23 determines that the instruction on the urine volume measurement is given by the user, and in a case where the user presses the second button D2 indicating that the urine volume measurement is not executed, through the input unit 24, the apparatus controller 23 determines that the instruction on the urine volume measurement is not given by the user. In a case where the apparatus controller 23 determines in Step S6 that the instruction on the urine volume measurement is given by the user, the process proceeds to Step S7.

In Step S7, the urine volume measurement unit 22 executes the urine volume measurement based on the ultrasound image U which includes the bladder B having the largest area, which is sent from the bladder extraction unit 20 in Step S3. Specifically, the urine volume measurement unit 22 measures the bladder diameter with respect to the bladder B in the ultrasound image U sent from the bladder extraction unit 20.

In subsequent Step S8, the constipation evaluation unit 18 causes the display unit 16 to display the result of the constipation evaluation in Step S2, and the urine volume measurement unit 22 causes the display unit 16 to display the result of the urine volume measurement in Step S7. For example, as shown in Fig. 5, the urine volume measurement unit 22 may superimpose and display the measurement result M of the bladder diameter on the ultrasound image U which includes the bladder B having the largest area, as the result of the urine volume measurement. Further, although not shown, for example, the constipation evaluation unit 18 may cause the display unit 16 to display the result of the constipation evaluation, together with the ultrasound image U on which the measurement result M of the bladder diameter is superimposed and displayed.

Thus, in a case where the urine volume measurement of the subject is performed, the user does not need to set the transmission/reception conditions of the ultrasonic waves focusing on the bladder B, and the urine volume measurement is smoothly performed by using the ultrasound image U acquired upon the constipation evaluation. Therefore, the effort of the user upon the urine volume measurement can be reduced and the time required for the urine volume measurement focused on the constipation evaluation can be shortened.

In this way, when the result of the constipation evaluation and the result of the urine volume measurement are displayed on the display unit 16, the operation of the ultrasound diagnostic apparatus 1 ends.

Further, in a case where determination is made in Step S4 that the ultrasound image from which the bladder is extracted is not present in the series of ultrasound images acquired in Step S1, and in a case where determination is made in Step S6 that the instruction on the urine volume measurement is not given by the user, each of the processes proceeds to Step S9 without the urine volume measurement.

In Step S9, the constipation evaluation unit 18 displays the result of the constipation evaluation in Step S2 on the display unit 16. For example, although not shown, the constipation evaluation unit 18 may superimpose and display the result of the constipation evaluation on the ultrasound image in which the rectum is captured. In this way, when the result of the constipation evaluation is displayed on the display unit 16, the operation of the ultrasound diagnostic apparatus 1 ends.

Here, in the examination of the lower abdomen of the subject, such as constipation evaluation and urine volume measurement, in order to reduce the burden on the subject, the user is required to perform the examination of the subject within a limited time.

With the ultrasound diagnostic apparatus 1 according to the embodiment of the present invention, the constipation evaluation unit 18 that performs constipation evaluation based on the ultrasound image acquired by the image acquisition unit 17 and a urine volume measurement availability determination unit 21 that determines whether or not urine volume measurement is available, from the ultrasound image U acquired by the image acquisition unit 17 in accordance with the transmission/reception condition of the ultrasonic wave which is used to perform the constipation evaluation by the constipation evaluation unit 18 are provided. Therefore, in a case where the urine volume measurement of the subject is performed, the user does not need to set the transmission/reception condition of the ultrasonic wave focusing on the bladder B, and the urine volume measurement is smoothly performed by using the ultrasound image U acquired upon the constipation evaluation. Accordingly, the effort of the user can be reduced, and the urine volume measurement can be easily performed regardless of the skill level of the user.

Note that, in Fig. 5, the horizontal length of the bladder B is measured as the bladder diameter, but the urine volume measurement unit 22 may measure the vertical length of the bladder B along the vertical line passing through the central part of the bladder B, as the bladder diameter. Further, the urine volume measurement unit 22 may also measure both the horizontal length and the vertical length of the bladder B as the bladder diameter.

In addition, in a case where the urine volume of the subject is measured, the bladder diameters are measured along three axes orthogonal to one another with the center of the bladder B of the subject set as the origin, respectively and the volume of the bladder B is calculated as the urine volume of the subject based on the measured three bladder diameters. Therefore, in a case where the urine volume measurement unit 22 measures the horizontal length and the vertical length of the bladder B and the measurement result is displayed on the display unit 16, for example, a guidance display prompting the user to image the tomographic plane of the bladder B, which is orthogonal to the tomographic plane of the bladder B to be represented by the ultrasound image in which the bladder diameters are measured, may be displayed on the display unit 16. Thereby, the user images the bladder B with a new tomographic plane using the transmission/reception condition of the ultrasonic wave for constipation evaluation, and the image acquisition unit 17 acquires a new ultrasound image. In this case, by using the newly acquired ultrasound image, for example, the urine volume measurement unit 22 may measure the horizontal length of the bladder B as a third bladder diameter, and calculate the volume of the bladder B from the measured three bladder diameters as the urine volume of the subject.

Further, the ultrasound diagnostic apparatus 1 may be a so-called stationary type that is not supposed to be carried, or a so-called portable type that can be easily carried. Here, the portable ultrasound diagnostic apparatus is often used in the field of home medical care, and many users are unfamiliar with the examination using the ultrasound diagnostic apparatus in the field of home medical care. The ultrasound diagnostic apparatus 1 according to the embodiment of the present invention can reduce the effort of the user and easily perform the urine volume measurement regardless of the skill level of the user. Accordingly, the ultrasound diagnostic apparatus 1 according to the embodiment of the present invention is particularly useful in a case of the portable type.

### Explanation of References

1: ultrasound diagnostic apparatus
2: ultrasound probe
3: diagnostic apparatus body
11: transducer array
12: transmission unit
13: reception unit
14: image generation unit
15: display controller
16: display unit
17: image acquisition unit
18: constipation evaluation unit
19: image memory
20: bladder extraction unit
21: urine volume measurement availability determination unit
22: urine volume measurement unit
23: apparatus controller
24: input unit
25: storage unit
26: processor
27: amplification unit
28: AD conversion unit
29: beamformer
30: signal processing unit
31: DSC
32: image processing unit
B: bladder
C: stool
D1: first button
D2: second button
G: guidance display
M: measurement result
U: ultrasound image

## Claims

1. An ultrasound diagnostic apparatus (1) comprising:
an ultrasound probe (2);
an image acquisition unit (17) that acquires an ultrasound image (U) of a subject by performing transmission and reception of an ultrasound beam to and from the ultrasound probe (2) in accordance with a predetermined transmission/reception condition;
a display unit (16) that displays the ultrasound image (U) acquired by the image acquisition unit (17);
a constipation evaluation unit (18) that performs constipation evaluation based on the ultrasound image (U) acquired by the image acquisition unit (17); and
a urine volume measurement availability determination unit (21) that determines whether or not urine volume measurement is available from the ultrasound image (U) acquired by the image acquisition unit (17) in accordance with the predetermined transmission/reception condition, wherein said ultrasound image is used to perform the constipation evaluation by the constipation evaluation unit.

2. The ultrasound diagnostic apparatus (1) according to claim 1, further comprising:
a bladder extraction unit (20) that extracts a bladder (B) based on the ultrasound image (U) acquired by the image acquisition unit (17),
wherein in a case where an ultrasound image from which the bladder (B) is extracted by the bladder extraction unit (20) is present in a series of the ultrasound images acquired by the image acquisition unit (17) in accordance with the predetermined transmission/reception condition which is used to perform the constipation evaluation by the constipation evaluation unit (18), the urine volume measurement availability determination unit (21) determines that the urine volume measurement is available.

3. The ultrasound diagnostic apparatus (1) according to claim 2,
wherein in a case where the urine volume measurement availability determination unit (21) determines that the urine volume measurement is available, the urine volume measurement availability determination unit (21) displays the ultrasound image (U) from which the bladder (B) is extracted by the bladder extraction unit (20) and a guidance display (G) prompting a user to give an instruction on whether or not to carry out the urine volume measurement, on the display unit (16).

4. The ultrasound diagnostic apparatus (1) according to claim 3, further comprising:
an input unit (24) that is used for the user to perform input operation.

5. The ultrasound diagnostic apparatus (1) according to claim 4, further comprising:
a urine volume measurement unit (22) that performs the urine volume measurement based on the ultrasound image (U) from which the bladder (B) is extracted by the bladder extraction unit (20).

6. The ultrasound diagnostic apparatus (1) according to claim 5,
wherein in a case where the user gives the instruction on carrying out the urine volume measurement through the input unit (24), the urine volume measurement unit (11) measures a bladder diameter, based on an ultrasound image which includes a bladder having a largest area among a plurality of the ultrasound images from which the bladder (B) is extracted by the bladder extraction unit (20), and displays the ultrasound image (U) which includes the bladder (B) having the largest area and a measurement result of the bladder diameter, on the display unit (16).

7. A computer implemented control method of the ultrasound diagnostic apparatus (1) according to claims 1 to 6, comprising:
acquiring an ultrasound image (U) of a subject by performing transmission and reception of an ultrasound beam to and from the ultrasound probe (2) in accordance with a predetermined transmission/reception condition;
displaying the acquired ultrasound image;
performing constipation evaluation based on the acquired ultrasound image (U); and
determining whether or not urine volume measurement is available from the ultrasound image (U) acquired in accordance with the predetermined transmission/reception condition, wherein said ultrasound image is used to perform the constipation evaluation.

## Patentansprüche

1. Ein Ultraschall-Diagnosegerät (1), das Folgendes umfasst:
eine Ultraschallsonde (2);
eine Bilderfassungseinheit (17), die ein Ultraschallbild (U) eines Subjekts erfasst, indem sie ein Senden und Empfangen eines Ultraschallstrahls zu und von der Ultraschallsonde (2) in Übereinstimmung mit einer vorbestimmten Sende-/Empfangsbedingung durchführt;
eine Anzeigeeinheit (16), die das von der Bilderfassungseinheit (17) erfasste Ultraschallbild (U) anzeigt;
eine Verstopfungsbewertungseinheit (18), die eine Verstopfungsbewertung auf der Grundlage des von der Bilderfassungseinheit (17) erfassten Ultraschallbildes (U) durchführt; und
eine Einheit (21) zur Bestimmung der Verfügbarkeit der Urinvolumenmessung, die aus dem Ultraschallbild (U), das von der Bilderfassungseinheit (17) in Übereinstimmung mit der vorbestimmten Sende-/Empfangsbedingung erfasst wurde, bestimmt, ob die Urinvolumenmessung verfügbar ist oder nicht, wobei das Ultraschallbild verwendet wird, um die Verstopfungsbewertung durch die Verstopfungsbewertungseinheit durchzuführen.

2. Das Ultraschall-Diagnosegerät (1) nach Anspruch 1, ferner umfassend:
eine Blasenextraktionseinheit (20), die eine Blase (B) auf der Grundlage des von der Bilderfassungseinheit (17) erfassten Ultraschallbildes (U) extrahiert,
wobei in einem Fall, in dem ein Ultraschallbild, aus dem die Blase (B) durch die Blasenextraktionseinheit (20) extrahiert wird, in einer Serie der Ultraschallbilder vorhanden ist, die durch die Bilderfassungseinheit (17) in Übereinstimmung mit der vorbestimmten Sende-/Empfangsbedingung erfasst werden, die verwendet wird, um die Verstopfungsbewertung durch die Verstopfungsbewertungseinheit (18) durchzuführen, die Urinvolumenmessverfügbarkeitsbestimmungseinheit (21) bestimmt, dass die Urinvolumenmessung verfügbar ist.

3. Das Ultraschall-Diagnosegerät (1) nach Anspruch 2,
wobei in einem Fall, in dem die Einheit (21) zur Bestimmung der Verfügbarkeit der Urinvolumenmessung feststellt, dass die Urinvolumenmessung verfügbar ist, die Einheit (21) zur Bestimmung der Verfügbarkeit der Urinvolumenmessung das Ultraschallbild (U), aus dem die Blase (B) durch die Blasenextraktionseinheit (20) extrahiert wird, und eine Anleitungsanzeige (G), die einen Benutzer auffordert, eine Anweisung zu geben, ob die Urinvolumenmessung durchgeführt werden soll oder nicht, auf der Anzeigeeinheit (16) anzeigt.

4. Das Ultraschall-Diagnosegerät (1) nach Anspruch 3, ferner umfassend:
eine Eingabeeinheit (24), mit der der Benutzer Eingaben vornehmen kann.

5. Das Ultraschall-Diagnosegerät (1) nach Anspruch 4, ferner umfassend:
eine Einheit zur Messung des Urinvolumens (22), die die Messung des Urinvolumens auf der Grundlage des Ultraschallbildes (U) durchführt, aus dem die Blase (B) durch die Blasenextraktionseinheit (20) extrahiert wird.

6. Das Ultraschall-Diagnosegerät (1) nach Anspruch 5,
wobei in einem Fall, in dem der Benutzer die Anweisung zur Durchführung der Urinvolumenmessung über die Eingabeeinheit (24) gibt, die Urinvolumenmesseinheit (11) einen Blasendurchmesser misst, basierend auf einem Ultraschallbild, das eine Blase mit einer größten Fläche unter einer Vielzahl der Ultraschallbilder enthält, aus denen die Blase (B) durch die Blasenextraktionseinheit (20) extrahiert wird, und das Ultraschallbild (U), das die Blase (B) mit der größten Fläche enthält, und ein Messergebnis des Blasendurchmessers auf der Anzeigeeinheit (16) anzeigt.

7. Computerimplementiertes Steuerungsverfahren für das Ultraschall-Diagnosegerät (1) nach einem der Ansprüche 1 bis 6, umfassend:
Erfassen eines Ultraschallbildes (U) eines Subjekts durch Durchführen des Sendens und Empfangens eines Ultraschallstrahls zu und von der Ultraschallsonde (2) in Übereinstimmung mit einer vorbestimmten Sende-/Empfangsbedingung;
Anzeige des erfassten Ultraschallbildes;
Bewertung der Verstopfung auf der Grundlage des erfassten Ultraschallbildes (U); und
Bestimmen, ob die Messung des Urinvolumens verfügbar ist oder nicht , aus dem Ultraschallbild (U), das in Übereinstimmung mit der vorbestimmten Sende-/Empfangsbedingung aufgenommen wurde, wobei das Ultraschallbild verwendet wird, um die Verstopfungsbewertung durchzuführen.

## Revendications

1. Appareil de diagnostic par ultrasons (1) comprenant :
une sonde à ultrasons (2) ;
une unité d'acquisition d'images (17) qui acquiert une image ultrasonore (U) d'un sujet en effectuant la transmission et la réception d'un faisceau ultrasonore vers et depuis la sonde ultrasonore (2) conformément à une condition de transmission/réception prédéterminée ;
une unité d'affichage (16) qui affiche l'image ultrasonore (U) acquise par l'unité d'acquisition d'images (17) ;
une unité d'évaluation de la constipation (18) qui effectue une évaluation de la constipation sur la base de l'image ultrasonore (U) acquise par l'unité d'acquisition d'images (17) ; et
une unité de détermination de la disponibilité de la mesure du volume d'urine (21) qui détermine si la mesure du volume d'urine est disponible à partir de l'image ultrasonore (U) acquise par l'unité d'acquisition d'images (17) conformément à la condition de transmission/réception prédéterminée dans laquelle ladite image ultrasonore est utilisée pour effectuer l'évaluation de la constipation par l'unité d'évaluation de la constipation.

2. L'appareil de diagnostic par ultrasons (1) selon la revendication 1, comprenant en outre :
une unité d'extraction de la vessie (20) qui extrait une vessie (B) sur la base de l'image ultrasonore (U) acquise par l'unité d'acquisition d'images (17),
dans le cas où une image ultrasonore à partir de laquelle la vessie (B) est extraite par l'unité d'extraction de la vessie (20) est présente dans une série d'images ultrasonores acquises par l'unité d'acquisition d'images (17) conformément à la condition de transmission/réception prédéterminée qui est utilisée pour effectuer l'évaluation de la constipation par l'unité d'évaluation de la constipation (18), l'unité de détermination de la disponibilité de la mesure du volume d'urine (21) détermine que la mesure du volume d'urine est disponible.

3. L'Appareil de diagnostic par ultrasons (1) selon la revendication 2,
dans le cas où l'unité de détermination de la disponibilité de la mesure du volume d'urine (21) détermine que la mesure du volume d'urine est disponible, l'unité de détermination de la disponibilité de la mesure du volume d'urine (21) affiche sur l'unité d'affichage (16) l'image échographique (U) à partir de laquelle la vessie (B) est extraite par l'unité d'extraction de la vessie (20) et un affichage de guidage (G) invitant l'utilisateur à donner une instruction sur la réalisation ou non de la mesure du volume d'urine.

4. L'appareil de diagnostic par ultrasons (1) selon la revendication 3, comprenant en outre :
une unité de saisie (24) utilisée par l'utilisateur pour effectuer des opérations de saisie.

5. L'appareil de diagnostic par ultrasons (1) selon la revendication 4, comprenant en outre :
une unité de mesure du volume d'urine (22) qui effectue la mesure du volume d'urine sur la base de l'image ultrasonore (U) à partir de laquelle la vessie (B) est extraite par l'unité d'extraction de la vessie (20).

6. L' Appareil de diagnostic par ultrasons (1) selon la revendication 5,
dans lequel, lorsque l'utilisateur donne l'instruction d'effectuer la mesure du volume d'urine par l'intermédiaire de l'unité d'entrée (24), l'unité de mesure du volume d'urine (11) mesure le diamètre de la vessie, sur la base d'une image ultrasonore qui comprend une vessie ayant la plus grande surface parmi une pluralité d'images ultrasonores dont la vessie (B) est extraite par l'unité d'extraction de la vessie (20), et affiche l'image ultrasonore (U) qui comprend la vessie (B) ayant la plus grande surface et un résultat de mesure du diamètre de la vessie, sur l'unité d'affichage (16).

7. Méthode de commande mise en oeuvre par ordinateur de l'appareil de diagnostic par ultrasons (1) selon les revendications 1 à 6, comprenant :
acquérir une image ultrasonore (U) d'un sujet en effectuant la transmission et la réception d'un faisceau ultrasonore vers et depuis la sonde ultrasonore (2) conformément à une condition de transmission/réception prédéterminée ;
l'affichage de l'image ultrasonore acquise ;
évaluer la constipation sur la base de l'image échographique acquise (U) ; et
déterminer si la mesure du volume d'urine est disponible , à partir de l'image ultrasonore (U) acquise conformément à la condition de transmission/réception prédéterminée dans laquelle ladite image ultrasonore est utilisée pour effectuer l'évaluation de la constipation.
